# EUROPÄISCHE PATENTSCHRIFT

(11) **EP 3 131 594 B1**
(45) Veröffentlichungstag und Bekanntmachung des Hinweises auf die Patenterteilung: **24.02.2021**
(21) Anmeldenummer: 15715168.9
(22) Anmeldetag: 10.04.2015
(51) Int. Cl.: A61M 1/00, A61B 1/00, A61B 17/42

(54) **DRAINAGE UND UNTERDRUCKPUMPE ZUR INTRAUTERINEN UNTERDRUCKTHERAPIE**
DRAINAGE SYSTEM AND VACUUM PUMP FOR INTRAUTERINE VACUUM THERAPY
DRAINAGE ET POMPE À DÉPRESSION POUR UNE THÉRAPIE INTRA-UTÉRINE PAR DÉPRESSION

(30) Priorität: 16.04.2014 DE 102014005679
(43) Veröffentlichungstag der Anmeldung: 22.02.2017
(73) Patentinhaber: Lohmann & Rauscher GmbH & Co. KG, 56567 Neuwied (DE)
(72) Erfinder: LOSKE, Gunnar, 22926 Ahrensburg (DE)
(74) Vertreter: Seranski, Klaus
(86) Internationale Anmeldenummer: PCT/EP2015/000756
(87) Internationale Veröffentlichungsnummer: WO 2015/158422

(56) Entgegenhaltungen:
- WO-A1-2012/123414
- DE-A1-102011 013 743
- DE-A1-102012 003 129
- DE-A1-102012 023 061
- US-A1- 2013 245 581

## Beschreibung

### Stand der Technik

Die Unterdrucktherapie (Vakuumtherapie, Vakuumschwammtherapie, Niederdrucktherapie) wird zur Behandlungen von äußeren Wunden eingesetzt. Ein offenporiger Polyurethanschaum oder ein anderer offenporiger Wundfüller (z.B. eine Baumwollgaze) werden in die Wunde eingelegt und mit einer Folie okkludiert. Anschließend wird an diesen Verband mit einem vakuumerzeugenden System (beispielsweise einer elektronischen Unterdruckpumpe) ein Unterdruck angelegt. Wundsekret und -ödem lassen sich auf diese Weise dauerhaft Ober einige Tage absaugen und infizierte Wunden können sich reinigen. Die Durchblutung wird verbessert, die Granulation angeregt. Ziel ist es, eine stabile sekundäre Wundsituation zu konditionieren, unter welcher die Wunde weiter abheilen kann. Die offenporigen Wundfüller können antimikrobiell ausgerüstet werden. Zusätzlich zur Okklusion kann der Verband auch kontrolliert gespült werden. Hierzu werden spezielle Pumpen verwandt, die sowohl saugen als auch mit einer Spüllösung spülen können.

Das Behandlungsprinzip wird auch endoskopisch beispielsweise zur Behandlung von Anastomosenleckagen am Enddarm oder der Speiseröhre innerlich angewandt.

Ebenso wird die Behandlung intraabdominell zur Therapie von Bauchfellendzündungen angewandt. Hierbei kommen auch offenporige Spezialfolien, an die ein Unterdruck angelegt werden kann, zum Einsatz. Ebenso gibt es spezielle Wundpflasterverbände, an die ein Unterdruck angelegt werden kann.

Intrauterine Behandlungsanwendungen der Vakuumtherapie sind in der US 2013/0245581 A1 beschrieben. Die DE 10 2012 003 129 A1 offenbart Drainagen und Platzierungssysteme zur endoskopischen Vakuumtherapie, bei denen in einem Schwammkörper ein Tubus, welcher am proximalen Ende eine trichterartige Aufweitung aufweist, vorgesehen sein kann. In dem Tubus kann ein Legestab eingelegt sein. Im Legestab kann ein Führungsdraht eingebracht sein. Auf dem Legestab der bekannten Platzierungssysteme kann ein Pusher aufgesetzt sein.

Der Erfindung liegt die Aufgabe zugrunde, eine zur intrauterinären Unterdrucktherapie einsetzbare Drainage bereitzustellen, die einfach verschiebbar ist.

Die Aufgabe wird durch die Merkmale des unabhängigen Patentanspruchs 1 gelöst.

Bevorzugte Ausführungsformen der Erfindung sind in den abhängigen Patentansprüchen angegeben.

### Erfindung

Bei der Erfindung handelt es sich um eine Drainage und elektronische Unterdruckpumpe, welche intrauterin zur Vakuumtherapie genutzt werden können.

Im Folgenden werden die Voraussetzungen und klinischen Problemdarstellungen beschrieben, aus denen sich die Ausführungen, auf denen die Erfindung beruht, ableiten.

### Atoner Uterus, uterine postpartale Blutung

Bei einer Schwangerschaft wächst sowohl der kräftige Muskelmantel des Uterus, die Plazenta als auch die Gebärmutterhöhle in der sich das Kind und Mutterkuchen befinden. Nach der Geburt löst sich aus der Gebärmutterhöhle der Mutterkuchen und es entsteht eine große intrauterine Wundfläche. Im Normalfall verschließen sich die Blutgefäße dieser Wundfläche durch die Kontraktion des Uterus, begleitet von hormonellen Einflüssen, und die Geburtsblutung sistiert. Im Fall einer Unfähigkeit des Uterus zur Kontraktion kann es zur sogenannten Atonie mit schwersten Blutungskomplikationen, bis hin zur Verblutung der Patientin kommen. Die konservativen Therapien bestehen in der manuellen Kompression und medikamentöser, hormoneller Stimmulation zur Kontraktion des Uterus. Die Muskulatur der Gebärmutter soll zur Eigenkontraktion angeregt werden. Beim Versagen der Therapie und Persistieren der Blutung muss als Ultima ratio die notfallmäßige operative Entfernung der blutenden Gebärmutter zur Lebensrettung der Mutter erfolgen.

### Endometritis, Infektionen nach der Geburt, Abort oder Schwangerschaftsabbruch

Unter einer Schwangerschaft kann es zu einer Infektion der Fruchthöhle mit Infektion des Fruchtwassers, Plazenta und Kindes kommen. Das Kind kann intrauterin versterben. Ober die Gebärmutterhöhle können Keime und Endotoxine in den mütterlichen Kreislauf eingeschwemmt werden und zur systemischen Infektion der Mutter führen. Lokal intrauterin besteht eine Entzündung des Endometriums, eine Endometritis.

Bei jedem Abort und nach einem Schwangerschaftsabbruch kann es zu einer von der inneren Wundfläche der Gebärmutter ausgehenden Infektion der Patientin kommen. Die Behandlung besteht in antimikrobieller Therapie und Kürettage. Des Weiteren kann eine transmurale Verletzung der Gebärmutterwand auftreten (iatrogen oder unter der Geburt).

Zusammengefasst können zwei wesentliche Probleme im Rahmen einer Schwangerschaft auftreten, bei welchen die Prinzipien der Vakuumtherapie anzuwenden wären: intrauterine Blutung und Infektion.

### Erfindung

### Fluidkommunikationselement

Ein offenporiges Fluidsammelelement ist mit mindestens einem Fluidkommunikationselement fluidleitend verbunden. Es können Flüssigkeiten und/oder Gase geleitet werden. Das Fluidkommunikationselement besteht aus einem Drainageschlauch, welcher mit seinem distalen Ende über fluidleitende Perforationsöffnungen mit dem Fluidsammelelement fest verbunden ist. Die Befestigung erfolgt Ober Nähte und/oder Klebung und/oder einer anderen Befestigungsart. Das Fluidkommunikationselement liegt in der Längsachse oder parallel zur Längsachse. Das Fluidkommunikationselement ist im Fluidsammelelement mit mehreren astartigen Abzweigungen versehen, die bis zu jeglichem Ort im Sammelelement führen können. Die Abzweigungen sind T-stückartig. Insbesondere verläuft das Fluidkommunikationselement wie eine Schlaufe in dem Fluidsammelelement. Es tritt am proximalen Ende ein, verläuft in einer Schlaufe durch den Körper und tritt am proximalen Ende aus. Das ein- und austretende Fluidkommunikationselement kann vorteilhaft y-förmig miteinander fluidleitend vereinigt werden. Die im Fluidsammelelement verzweigten und/oder schlaufenförmigen und/oder geschlängelten und/oder bogenförmigen und/oder spiralig verlaufenden Fluidkommunikationselemente haben den Vorteil, dass bereits durch den Verlauf eine ausreichende Fixierung im Fluidsammelelement vorliegt und keine zusätzliche Naht oder Klebung notwendig ist. Das Fluidkommunikationselement kann in sich unterschiedliche Lumina haben. Es kann mit den Aufzweigungen bis an die Oberfläche des Fluidsammelelementes geführt werden und auch zur Spülung der Oberfläche und/oder des Körpers genutzt werden.

Mit seinem proximalen Ende ist es mit einem vakuumerzeugenden System verbunden. Dieses besteht insbesondere aus einer Vakuumpumpe, mit welcher ein Unterdruck zwischen 20 und 200 mmHg erzeugt werden kann. Der Schlauch ist unterdruckstabil. Er ist vorzugsweise durchsichtig. Er hat bevorzugt einen Durchmesser zwischen 2 mm und 20 mm und eine Länge von 30 cm bis 120 cm.

### Fluidsammelelement

Das Fluidsammelelement besteht bevorzugt aus einem offenporigen Polyurethanschaumkörper. Es besteht aus einem anderen Material, welches fluidsammelnde offenporige Eigenschaften hat. Es besteht aus einem und/oder mehreren Kunststoffen. Es besteht aus einer Kombination von verschiedenen offenporigen Materialien. Insbesondere besteht es aus einem offenporigen Schaum und einer offenporigen Folie. Bevorzugt liegen die Poren dicht an dicht wie bei einem Schwammkörper. Die Porengröße ist bevorzugt zwischen 100 µm und 2000 µm.

Das Fluidsammelelement kann über jegliche Körperform verfügen. Bevorzugt ist die Körperform, eine dem Uterus nachempfundene Birnenform. Die Körperform ist bevorzugt eine zylindrische Form. Die Köperform ist bevorzugt eine Eiform. Die Größe des Körpers ist der Größe des Uteruslumen nach dem Fortschritt der Schwangerschaft angepasst. Sie liegt in mehreren stufenweisen angepassten Größen vor. Der quere Durchmesser des Körpers in der Querachse misst bevorzugt von 1 cm bis 15 cm. Die Länge des Körpers in der Längsachse beträgt bevorzugt 2 cm bis 30 cm. Das Fluidsammelelement kann in der Größe zugeschnitten werden. Das Fluidsammelelement ist so zu bemessen, dass es vollständig in die Gebärmutter eingeführt werden kann.

Die Erfindung wird intrauterin angewandt. Transvaginal wird es über den eröffneten Gebärmuttermund das Fluidsammelelement in die Gebärmutterhöhle eingeführt. Der Gebärmuttermund kann hierzu bei Bedarf dilatiert werden. Nach einer Geburt ist der Gebärmuttermund eröffnet und hierdurch ausreichend weit. Eine Dilatation ist dann nicht erforderlich. Nach der intrauterinen Platzierung wird an das Fluidkommunikationselement ein Unterdruck angelegt. Die Gebärmutter kollabiert unter dem Sog und wird an das Fluidsammelelement angesogen.

Hierdurch soll die Eigenkontraktion der Gebärmutter, die bei der Atonie versagt, mechanisch angeregt werden. Durch den angelegten Unterdruck wird die Kontraktion der Gebärmutter induziert und die körpereigenen Blutstillungsmechanismen unterstützt. Wird ein Unterdruck an das Fluidsammelelement angelegt, legt sich die Innenwand der Gebärmutter an die offenporige Oberfläche des Fluidsammelelementes und wird angesaugt. Auch der Gebärmuttermund, über welchen eine Verbindung über die Vagina nach außen besteht und von hier Luft angesaugt werden könnte, legt sich an den Fluidsammelkörper an. Durch das Ansaugen des Gewebes bildet sich ein Kompartiment, in welchem der Unterdruck erzeugt werden kann.

Wenn das Fluidsammelelement noch in die Vagina ragt, kann Luft von außen durch den Introitus vaginae angesaugt werden und den Sogaufbau in der Gebärmutter verhindern. Die Abdichtung der Vagina kann zusätzlich mittels eines Pessars, welches über eine Perforationsöffnung über den Drainageschlauch geführt wird, oder einem andersartigen vaginalen Verschluss mittels einer nicht fluidleitenden Vaginaltamponade erreicht werden. Diese sitzt sozusagen wie ein Korken in der Vagina und dichtet diese zum Fluidsammelelement ab.

Am proximalen Ende kann das Fluidsammelelement auch nicht fluidleitend ausgerüstet sein, um bei einer vaginalen Platzierung eine bessere Abdichtung zur Vagina zu schaffen. Es kann auch die Wand des Fluidkommunikationselementes an seinem Austrittsort am Fluidsammelelement kolbig aufgetrieben sein und sozusagen als Stöpsel oder Korken zum Verschluss der Vagina beitragen, damit keine Luft angesaugt wird.

Bei einer Vakuumtherapie wird mit dem Unterdruck sowohl eine Sogkraft, die von der Wundfläche weggewandt ist, als auch mit dem angesaugten Fluidsammelelement auch eine Druckkraft, die auf die Wundfläche hin gerichtet ist, ausgeübt. Die Druckkraft auf eine Wundoberfläche ist abhängig von der Höhe des Unterdrucks. Sie ist auch abhängig von dem Maß der Ausfüllung der Wunde mit dem Sammelelement und von der Komprimierbarkeit des Fluidsammelelements. Je mehr das Lumen der Intrauterinhöhle mit dem Fluidsammelelement ausgefüllt ist, desto mehr der inneren Wundfläche hat Kontakt mit dem Fluidsammelelement. Es kann eine innere Tamponade mit dem Fluidsammelelement vorgenommen werden und anschließend zusätzlich der Unterdruck angelegt werden. Es können auch mehrere einzelne Fluidsammelelemente eingelegt werden. Diese zusätzlichen Elemente müssen nicht einzeln mit einem Fluidkommunikationselement ausgerüstet werden. Durch die offenporige Oberfläche ist es zur Vakuumerzeugung an dem Element ausreichend, wenn es mit der Oberfläche in direktem fluidleitenden Kontakt zu einem anderen Element steht, an welches ein Unterdruck angelegt werden kann. Je mehr das innere Lumen mit dem Fluidsammelelement tamponiert wird, desto größer ist die innere Wundfläche, die direkt dem Sog ausgesetzt ist. Wenn ein deutlich kleinvolumigeres Fluidsammelelement in der Gebärmutterhöhle platziert wird, ist der direkte Kontakt zum Fluidsammelelement geringer. Je weniger komprimierbar das Fluidsammelelement ist, desto mehr Druck wird bei der Vakuumentfaltung auf die angesaugte Wundfläche ausgeübt.

Dieser Effekt der mechanischen Druckausübung wird bei der intrauterinen Vakuumtherapie zur Blutstillung eingesetzt. Zum einen wird die Uteruswand an das Fluidsammelelement herangezogen und kontrahiert sich dabei um das Fluidsammelelement, zum anderen wird durch den Unterdruck ein mechanischer Druck von innen auf die Uteruswand ausgeübt. Je höher der Unterdruck angelegt wird, desto höher ist auch die Druckwirkung auf die Innenwand. Je mehr die Höhle austamponiert ist, desto höher ist die Druckwirkung auf die Innenwand.

Der offenporige Fluidsammelkörper kann von weicher oder fester Konsistenz sein, er kann unter einem Unterdruck komprimiert werden. Er kann seine Form unter einem Unterdruck beibehalten und unterdruckstabil sein. Das Maß der Unterdruckstabilität kann in Prozent zum Ursprungkörper ausgedrückt werden. Unterdruckstabilität von 100% bedeutet: der Körper verformt sich unter dem Unterdruck nicht. Unterdruckstabilität von 50% bedeutet: Unter einem Unterdruck verkleinert sich der Körper um die Hälfte, und so fort. Die Angaben der Unterdruckstabilität sollen für einen angelegten Unterdruck von -20 bis - 200 mmHg gelten. Analog kann die die Unterdruckstabilität auch durch den Wert der Komprimierbarkeit ausgedrückt werden.

Je kompressionsstabiler das Fluidsammelelement ist desto höher ist Druckwirkung auf die Innenwand. Bei einer innenliegenden Tamponade kann operativ oder durch die Bauchdecke durch äußeren Druck eine zusätzliche Kompression ausgeführt werden und zur Blutstillung beitragen. Insbesondere soll für das Fluidsammelelement eine Kompressionsstabilität von 10 -90% seines Ausgangsvolumens bei einem Sog zwischen 20 und 200 mmHg bestehen. Das bedeutet bei einem solchen Unterdruck verkleinert sich das Volumen des Fluidsammelelements auf 10 - 90% seines ursprünglichen Volumens.

Das Fluidsammelelement dient als Medikamententräger. Insbesondere ist das Fluidsammelelement mit blutstillenden Medikamenten ausgerüstet. Die blutstillenden Medikamente sind auf der Oberfläche des Fluidsammelelements aufgebracht und kommen in direktem Kontakt zur inneren Wundfläche. Die blutstillenden Medikamente können mineralisch sein. Sie können als Pulver und/oder Flüssigkeiten und/oder Schäume und/oder Gazen und/oder Salben auf das Fluidsammelelement gebracht werden. Sie können als Puder auch in die Höhle vor Platzierung des Fluidsammelelementes eingesprüht werden. Insbesondere können die blutstillenden Medikamente vor der Platzierung des Fluidsammelelementes endoskopisch in die Uterushöhle eingebracht werden. Die Medikamente können über den Arbeitskanal eines Endoskopes eingebracht werden. Sie können eingespült oder gesprüht werden. Sie können mit Überdruck eingeblasen werden.

Sie können Ober zusätzliche fluid- und/oder gasleitenden Kommunikationselemente, die vorzugsweise schlauchförmig sind und in dem Fluidsammelelement liegen, in die Uterushöhle eingebracht werden. Insbesondere sollen die Medikamente gezielt über eine offenporige und/oder einseitig offenporige Folie, mit welcher das Fluidsammelelement umhüllt ist und welche die Oberfläche des Fluidsammelelementkörpers darstellt, eingebracht werden. Die an der Oberfläche liegende offenporige und/oder einseitig offenporige Folie ist mit mindestens einem zusätzlichen Fluidkommunikationselement versehen, über welches Medikamente zugeführt werden können. Die Nutzung der Folie als Medikamententräger oder Zufuhrmedium hat den Vorteil, dass sich das Medikament nur auf und/oder innerhalb der Folie verteilt. Hierdurch kann an der inneren Wundfläche gezielt das Medikament aufgebracht werden. Die blutstillenden Medikamente können aus Enzyme, Mineralien, Gerinnungsfaktoren, Hormonen bestehen. Insbesondere können die Medikamente aus Zeolithen, mikroporösen kristalinen Aluminosilikaten oder Chitin und/oder Chitosan-Biopolymeren oder Smekit Aluminum-Silikat oder Kaolin Aluminium-Silikat oder Plasmaprotein bestehen.

### Führungsstab und Hülse

Die Platzierung des Fluidsammelelementes erfolgt transvaginal mit Hilfe eines Führungsstabes. Der Führungsstab besteht aus Metall oder Kunststoff. Es ist 20 cm -120 cm lang. Er hat einen Durchmesser von 2mm -25 mm. Am proximalen Ende ist er mit einem Griff ausgerüstet. Am distalen Ende endet er stumpf oder abgerundet. Das distale Ende ist stempelartig aufgetrieben geformt. Insbesondere ist der Führungsstab an seinem distalen Ende weich abknickbar und/oder flexibel und atraumatisch konstruiert. Der Führungsstab ist dem natürlichen Verlauf des Geburtskanals und Gebärmutter angepasst und gebogen.

Der Führungsstab wird in das Fluidsammelelement eingeführt. Das Fluidsammelelement weist in der Längsachse oder parallel zur Längsachse einen zylindrischen röhrenartigen Hohlraum auf zur Aufnahme des Führungsstabes auf. Der Durchmesser des Hohlraumes entspricht dem Durchmesser des Stabes. Der Durchmesser des Hohlraumes ist etwas geringer als der Durchmesser des Stabes, so dass sich dieser in dem Hohlraum verklemmen kann und hierdurch fixiert ist. Der röhrenartige Hohlraum endet blind in dem Fluidsammelelement. Bis zu diesem Ende wird der Führungsstab eingeführt.

Die zylinderförmige Aussparung zur Aufnahme des Führungsstabes kann aus einem im dem Fluidsammelelement integriertem Rohr bestehen. Das Rohr kann am distalen Ende blind verschlossen sein. Das Rohr kann sich über die gesamte Längsachse des Fluidsammelelementes erstrecken und beidseits offen sein. Das Rohr kann aus Kunststoff bestehen. Das Rohr ist am proximalen Ende trichterförmig geformt und schließt mit dem Fluidsammelelement bündig ab, oder überragt es. Das Rohr kann fluidleitend perforiert sein.

Das Rohr bzw. der röhrenartige Hohlraum enden am distalen Ende innerhalb des Fluidsammelelementes. Hierbei wird beim Vorschieben des Fluidsammelelementes die Perforationsgefahr des Uterus verringert.

Der röhrenartige Hohlraum reicht durch die gesamte Längsachse des Fluidsammelelementes, er hat am proximalen Endes des Fluidsammelelementes eine Eintrittsöffnung und am distalen Ende eine Austrittsöffnung. In diesem Fall kann das Fluidsammelelement auf dem Führungsstab hin und her geschoben werden. Zur Platzierung des Fluidsammelelementes wird zunächst der Führungsstab transvaginal in die Uterushöhle eingelegt und dann das auf dem Führungsstab befindliche Sammelelement nach distal transvaginal in die Uterushöhle eingeschoben.

Der Sog wird angelegt und der Führungsstab entfernt. Der Führungsstab kann im mittleren Anteil mit einem scheibenartigen Aufsatz versehen sein. Wenn der Stab in das Fluidelement eingeführt worden ist, liegt das proximale Ende des Fluidsammelelementes dem scheibenartigen Aufsatz an, so dass beim Vorschieben sowohl mit dem distalen Ende des Stabes als auch mit der scheibenartigen Begrenzung geschoben wird. Der scheibenartige Aufsatz kann nach distal auf dem Stab verschoben werden. Er ist mit einem weiteren Schiebestab, der in dem Führungsstab integriert ist und in einem Längsschlitz des Führungsstabes hin und her geschoben werden kann, verbunden. Beim Zurückziehmanöver des Führungsstabes, d.h. beim Entfernen aus dem Fluidsammelelement, kann gleichzeitig der scheibenartige Aufsatz nach vorn geschoben werden, so dass das Fluidsammelelement beim Entfernen des Führungsstabes in Position verbleibt.

Der Führungsstab ist mit einer Pusherhülse ausgerüstet, welche ebenfalls aus Metall oder Kunststoff besteht geführt werden. Bei dem Pusher handelt es sich um ein Rohr mit Öffnungen am distalen und proximalen Ende. Die Hülse hat einen etwas größeren Durchmesser als der Stab. Am distalen Ende ist die Hülse stempelartig geformt. Erfindungsgemäß schließt sie am distalen Ende mit einer Scheibe zum Schieben des Fluidsammelelementes abund/oder ist am distalen Ende trichterartig geformt. Die Hülse soll das proximale Ende des Fluidsammelelementes zur Platzierung vor sich her schieben. Die Hülse ist auf dem Führungsstab verschieblich. Mit der auf dem Führungsstab verschieblichen Hülse wird das Fluidsammelelement zur Platzierung nach distal verschoben. Wenn der Stab entfernt wird, fixiert die Hülse das Fluidsammelelemente in der Platzierungsposition. Die Hü Ise kann sowohl bei einem in dem Fluidsammelelement blind endenden röhrenartigen Hohlraum als auch bei einem beidseits offenen Hohlraum angewandt werden. Die Hülse hat einen Querdurchmesser von 3 mm bis 25 mm. Der scheibenartige Abschluss hat einen Durchmesser bis zu 5 cm. Die Hülse ist 20 cm bis 80 cm lang. Die Hülse hat am proximalen Ende einen Führungsgriff.

Insbesondere soll ein Endoskop als Führungsstab verwendet werden. Der Pusher kann auf dem Endoskop in Längsachse verschoben werden und gleiten. Über den Arbeitskanal des Endoskops lassen sich blutstillenden Medikamente vor der Platzierung der Drainage lokal in der Wundhöhle verabreichen. Es werden konventionelle Gastroskope und Koloskope als Führungsstäbe verwendet.

### Unterdruckpumpe

Anders als die Vakuumtherapie bei infizierten Wunden ist die intrauterine Vakuumtherapie bei der Uterusatonie primär auf eine kurzfristige Therapiedauer von wenigen Minuten oder einigen Stunden ausgelegt. Sie kann unterbrochen und bei Bedarf wiederaufgenommen werden.

Der Unterdruck für die intrauterine Therapie wird insbesondere von einer elektronischen Unterdruckpumpe erzeugt. Die Pumpe ist fluidleitend mit mindestens einem Sekretauffangbehälter ausgerüstet. Der Auffangbehälter hat bevorzugt eine Volumen von 100 - 1000 ml.

Die Unterdruckanwendung kann kontinuierlich und intermittierend erfolgen. Die Vakuumpumpe kann spezifische Sogmuster generieren. Hierzu ist die die Pumpe mit einer Steuerung ausgerüstet, welche sich Ober einen Anzeigebildschirm bedienen lässt. Die Pumpe misst Ober Drucksensoren kontinuierlich die Unterdruckwerte im unterdruckleitenden System und ist mit dem Fluidkommunikationselement fluidleitend verbunden. Der Unterdruck kann auch über elektronische Messfühler, die in dem Fluidkommunikationselement oder Fluidsammelelement integriert sind, an das Steuerelement zurückgekoppelt werden. Über das Steuerelement regelt die Pumpe permanent den Unterdruck automatisch nach, so wie er in den Voreinstellungen der Steuereinheit festgelegt wurde.

Auch das Eigenkontraktionsmuster der Gebärmutter kann an die Steuereinheit zurückgekoppelt werden. Das Sogmuster der Pumpe kann dem Eigenkontraktionsmuster des Uterus angepasst werden und dieses verstärken. Hierzu können direkte elektronische und/oder elektromyographische und/oder tonische Ableitungen des Uterus über Messfühler abgeleitet werden, die dem Steuerelement zugeführt werden. Die Kontraktionen der Gebärmutterwand können über Kontaktelektroden, die der Wand von innen oder außen anliegen, abgeleitet werden. Die Anpassung an das Eigenkontraktionsmuster erfolgt automatisch. Die Unterdrucktamponade wirkt auf diese Weise wie ein mechanischer Schrittmacher des Uterus und unterstützt und verstärkt die Eigenkontraktionsfähigkeit. Das Unterdruckmuster kann mit der Kontraktion des Uterus synchronisiert werden.

Mit der Steuerung lassen sich die Unterdruckwerte individuell einstellen. Die Dauer des Unterdrucks und Unterdruckpausen können gesteuert werden. Der minimale Unterdruck und der maximale Unterdruck, sowie Abstufungen zwischen den Werten können eingestellt werden. Das Anstiegs- und Abstiegsprofil und/oder die Geschwindigkeit der Unterdruckerzeugung kann eingestellt werden. Es lassen sich individuelle Druckprofile einstellen. Insbesondere können mit einer elektronisch kontrollierten Vakuumpumpe wehenartige Sogmuster erzeugt werden, die die natürlichen Kontraktionen des Uterus imitieren. Insbesondere hat das wehenförmige Saugprofil eine aktive Unterdruckphase von 10 sec bis 120 sec Sogdauer und eine Sogunterbrechungspause von 10 - 120 sec Dauer. Insbesondere kann das Sogprofil zwischen einem Basisunterdruck und Spitzenunterdruck pendeln. Es kann insbesondere um einen Mittleren Unterdruck undulierend pendeln. Der Basisunterdruck kann einem vollständigen Sogabfall auf 0 mmHg entsprechen.

Insbesondere soll der Basisunterdruck nicht einem Unterdruck von 0 mmHg entsprechen, sondern kontinuierlich niedriger als 0 mmHg sein, so dass der inneren Wundfläche unter der Therapie ein permanenter Unterdruck anliegt. Zusätzlich soll auf diesen permanenten Basisunterdruck ein ergänzender Unterdruck in Wehenform und/oder Sinuskurvenform und/oder Sägeblattform und/oder undulierender Wellenform mit oder ohne Pausen angelegt werden. Auch bei einem schwankenden Druckprofil soll immer ein Unterdruck an der inneren Wundfläche anliegt. Dieser Basisunterdruck soll bevorzugt zwischen -5 und -100 mmHg liegen. Mit diesem Unterdruckprofil soll eine postpartale Dauerkontraktion des Uterus mit ergänzenden verstärkenden wehenartigen Kontraktionen imitiert werden, die zur Blutstillung erforderlich ist. Die ergänzenden wehenartigen postpartalen Kontraktionen können unter einem kontinuierlichen Dauersog durch die Pumpe auch hormonell medikamentös und als elektrische Schrittmacherimpulse an die Uterusmuskulatur geleitet werden.

Die Kontraktionsmuster werden durch den Sog auf die Wände des Uterus übertragen. Insbesondere ist das Kontraktionsmuster wellenförmig verlaufend. Es ist insbesondere dem glockenförmigen Verlauf einer Sinuskurve folgend.

Die Pumpe soll ein sägezahnartiges Kontraktionsmuster erzeugen können. Die Pumpe soll nach einem Sogabfall bei einem Todvolumen von 100 - 1000ml in einem raschen Zeitintervall von weniger als 2 sec den maximalen Spitzenunterdruck erzeugen können. Insbesondere soll bei einem Todvolumen von 200 bis 500 ml in weniger als 2 sec der Spitzenunterdruck erzeugt werden können. Der Druckabfall auf den Basisunterdruck soll in einem längeren Intervall stattfinden können. Dieses Intervall soll zwischen 10 und 120 sec dauern. Umgekehrt soll auch der Sogaufbau allmählich in einem Zeitintervall von 10 sec bis 120 sec stattfinden und der Sogabfall auf den Basiswert rasch innerhalb von 2 sec bis 5 sec stattfinden können.

Insbesondere soll der Unterdruck Ober einen Zeitraum von 10 sec bis 120 sec erzeugt werden können und. Der Druckkurvenverlauf kann individuell eingestellt werden. Es können sowohl die Basisunterdruckwerte, unter welcher der Unterdruck nicht unterschreiten soll, als auch die zeitlichen Verläufe mit Dauer der Basis und/oder Spitzendrücke und/oder Abstufungen im Druckverlauf und/oder Geschwindigkeit des Unterdruckaufbaus und/oder Abbaus und/oder die Dauer des Druckplateaus und/oder Pausen individuell bestimmt werden und über das Eingabemodul der Pumpe eingestellt werden.

Die Vakuumtherapie bei der Atonie des Uterus dient in erster Linie dazu, die Atonie des Uterus zu überwinden und mechanisch durch den inneren Unterdruck die Gebärmutter aktiv zur Kontraktion zu bringen und hierdurch die natürliche Blutstillung zu induzieren oder zu unterstützen. Sobald dieser Mechanismus in Gang gesetzt wurde, ist auch eine frühzeitige Entfernung des Fluidsammelelementes möglich. Im Unterschied zu dieser kurzzeitigen Therapie wird die Vakuumtherapie zur Behandlung von infizierten Wunden über mehrere Tage angewandt und mehrfach im mehrtägigen Abstand gewechselt. Die Vakuumtherapie bei der Atonie kann auch über einen längeren Zeitraum von Tagen angewandt werden, dann wird der Schwamm beim Wechsel kontinuierlicher verkleinert und der Größe des Uteruslumen angepasst. Bei der Atonie wird die Vakuumtherapie als Notfallmaßnahme mit einem vollkommen neuen therapeutischen Ansatz zur Gebärmutterkontraktion genutzt.

Die Vakuumtherapie kann auch bei Infektionen des Uterus durchgeführt werden. Insbesondere ist die Vakuumtherapie zur Therapie der Endometritis postpartal, nach einem Abort oder einer Schwangerschaftsunterbrechung anwendbar.

Die vorbeschriebenen Ausführungen der Erfindung sind hierzu in gleicher Weise geeignet. Die Therapie wird nicht nichtkurzfristig Ober Stunden, sondern über mehrere Tage durchgeführt. Die Drainage wird nach einigen Tagen erneuert. Die Größe des Fluidsammelelementes ist der Größe des Uterusvolumens anzupassen. Bei Bedarf wird der Muttermund als Zugang zur Uterushöhle erweitert. Das Vakuum wird ebenfalls Ober ein elektronisches Pumpensystem erzeugt. Es werden Unterdrücke von -20 bis -200 mmHg angewandt. Es wird vorzugsweise ein kontinuierlicher Sog angewandt. Es wird ein intermittierender Sog angewandt. Der intermittierende Sog kann einem Wehenrhythmus nachempfunden sein. Es lassen sich die gleichen Druckmuster, wie vorbeschrieben verwendet werden. Die Drainage ist in endoskopischer Legetechnik platzierbar.

Eine weitere Indikation liegt bei der Perforation bzw. Ruptur des Uterus vor. Hierbei liegt ein Uteruswanddefekt vor. Durch die intrauterine Unterdruckanlage kann der Defekt abgedichtet werden und zusammengezogen werden und verkleben.

### Figuren

Im Folgenden wird die Erfindung anhand von Figuren, die Ausführungsbeispiele darstellen erläutert.

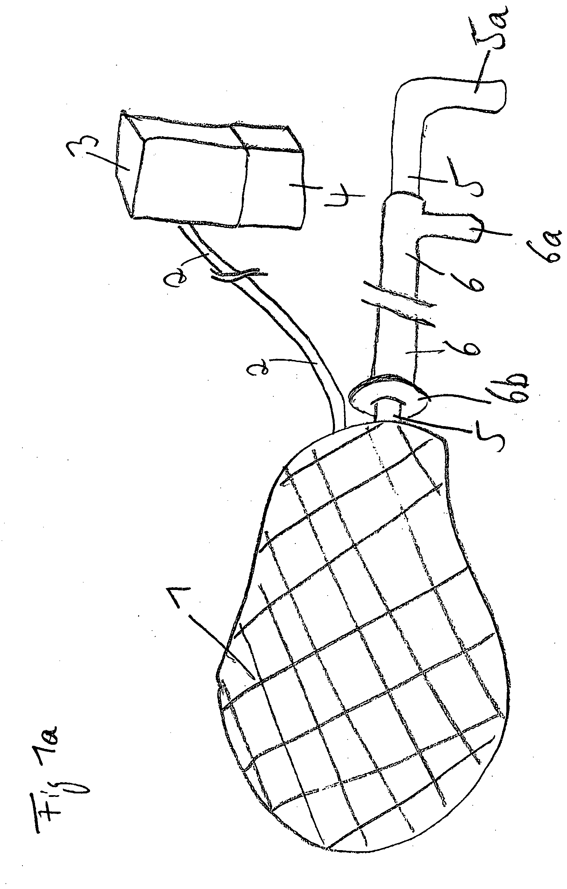

Figur 1a ist eine Aufsichtsdarstellung des Fluidsammelelement (1) in angedeuteter Birnenform. Dieses ist fluidleitend verbunden mit einem schlauchförmigen Fluidkommunikationselement (2), welches mit einem Sekretauffangbehälter (3) mit einer unterdruckerzeugenden Pumpe (4) fluidleitend verbunden ist. In das Sammelelement (1) ist ein Führungsstab (5) mit Handgriff (5a) eingeführt, welcher sich in einer auf ihm verschieblichen Hülse (6) befindet, diese ist am proximalen Ende auch mit einem Handgriff (6a) und endet am distalen Ende in einer scheibenartige Platte (6b) zum Vorschieben des Fluidsammelelementes (1).

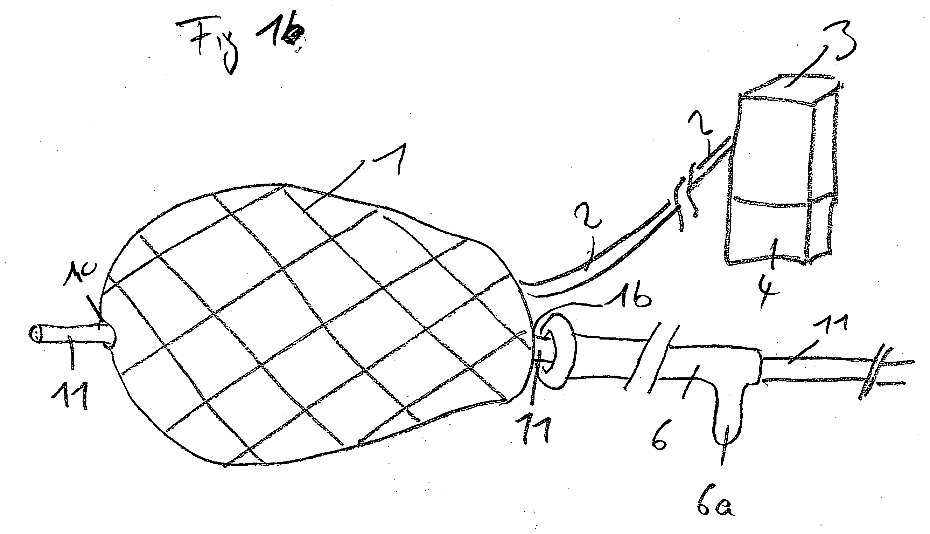

Figur 1b ist wie Figur 1 eine Aufsichtsdarstellung des Fluidsammelelement (1) in angedeuteter Birnenform. Dieses ist fluidleitend verbunden mit einem schlauchförmigen Fluidkommunikationselement (2), welches mit einem Sekretauffangbehälter (3) mit einer unterdruckerzeugenden Pumpe (4) fluidleitend verbunden ist. In das Sammelelement (1) ist ein Endoskop (11) hindurch geführt. Das Endoskop (11) tritt in das Fluidsammelelement (1) in einer Eintrittsöffnung (1b) ein und einer Austrittsöffnung (1c) aus. Das Endoskop (11) befindet sich in einer auf ihm verschieblichen Hülse (6) befindet, diese ist am proximalen Ende auch mit einem Handgriff (6a) und endet am distalen Ende in einer scheibenartige Platte (6b) zum Vorschieben des Fluidsammelelementes (1). Das Sammelelement ist auf dem Endoskop (11) verschieblich.

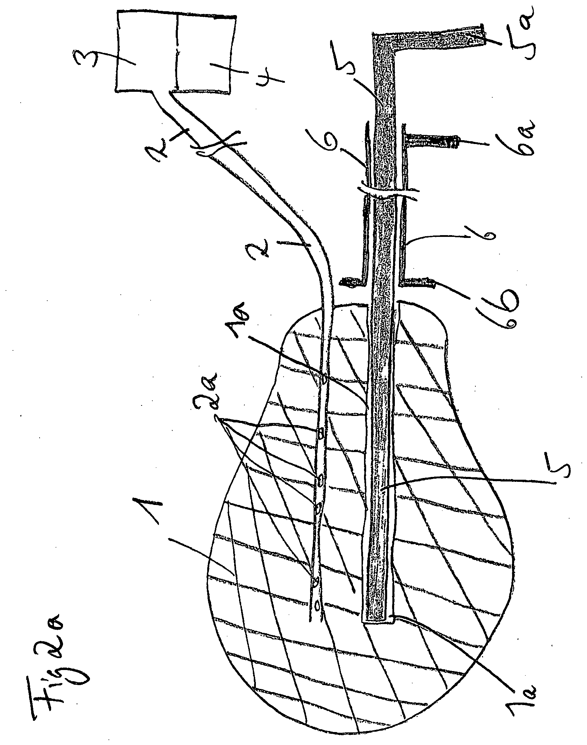

Figur 2a ist eine Längsschnittdarstellung von Figur 1. Das Fluidsammelelement (1) in angedeuteter Birnenform ist mit dem schlauchförmigen Fluidkommunikationselement (2) verbunden. Am distalen Ende ist das Fluidkommunikationselement mit seitlichen Perforationsöffnungen (2a) ausgerüstet. Das Fluidkommunikationselement (2) ist mit einem Sekretauffangbehälter (3) einer unterdruckerzeugenden Pumpe (4) fluidleitend verbunden. In das Sammelelement (1) ist eine zylinderförmige Aussparung (1a) in Längsrichtung vorhanden, in diese ist ein Führungsstab (5) mit Handgriff (5a) eingeführt, welcher sich in einer auf ihm verschieblichen Hülse (6) befindet, diese ist am proximalen Ende auch mit einem Handgriff (6a) und endet am distalen Ende in einer scheibenartige Platte (6b) zum Vorschieben des Fluidsammelelementes (1).

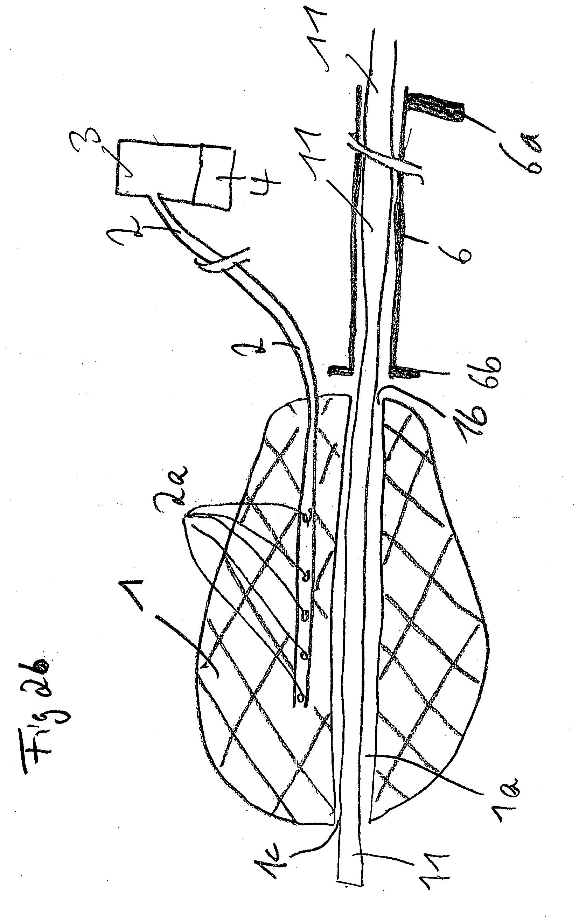

Figur 2b ist eine Längsschnittdarstellung von Figur 1a. Das Fluidsammelelement (1) in angedeuteter Birnenform ist mit dem schlauchförmigen Fluidkommunikationselement (2) verbunden. Am distalen Ende ist das Fluidkommunikationselement mit seitlichen Perforationsöffnungen (2a) ausgerüstet. Das Fluidkommunikationselement (2) ist mit einem Sekretauffangbehälter (3) einer unterdruckerzeugenden Pumpe (4) fluidleitend verbunden. In das Sammelelement (1) ist eine zylinderförmige Aussparungen (1a) in Längsrichtung vorhanden, in diese ist ein Endoskop (11) in die Eintrittsöffnung (Ib) eingeführt und durch die Austrittsöffnung (1c) ausgeführt. Auf dem Endoskop (11) befindet sich eine auf ihm verschiebliche Hülse (6), diese ist am proximalen Ende auch mit einem Handgriff (6a) und endet am distalen Ende in einer scheibenartige Platte (6b) zum Vorschieben des Fluidsammelelementes (1).

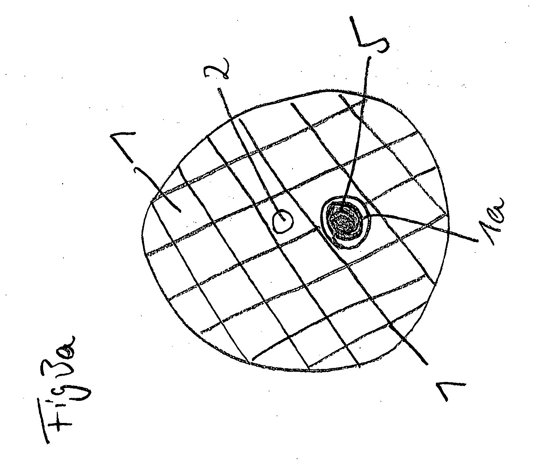

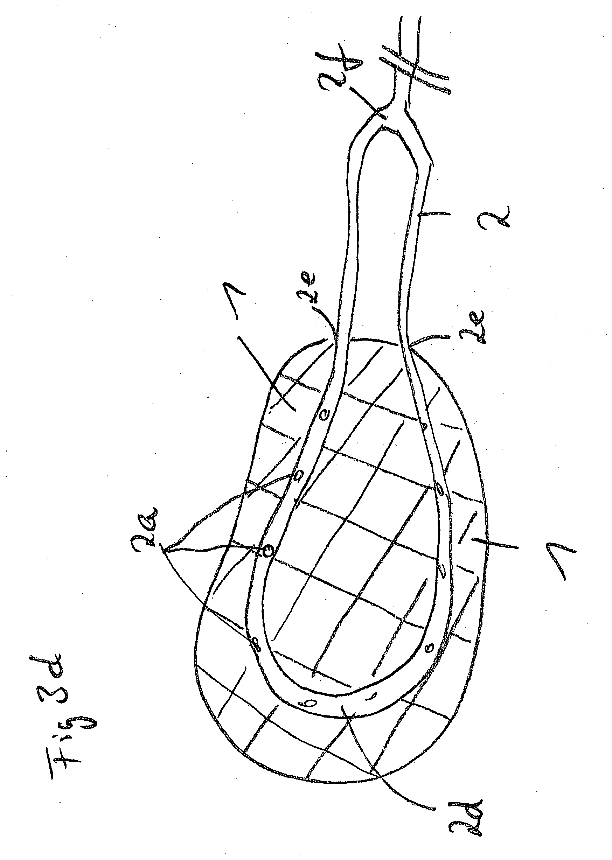

Figur 3a ist eine Querschnittdarstellung von Figur la. Man erkennt den Körper des Fluidsammelelementes (1) mit zylindrischer Aussparung (1a). In die Aussparung (1a) ist der Führungsstab (5) eingebracht. (2) ist das schlauchförmige Fluidkommunikationselement.

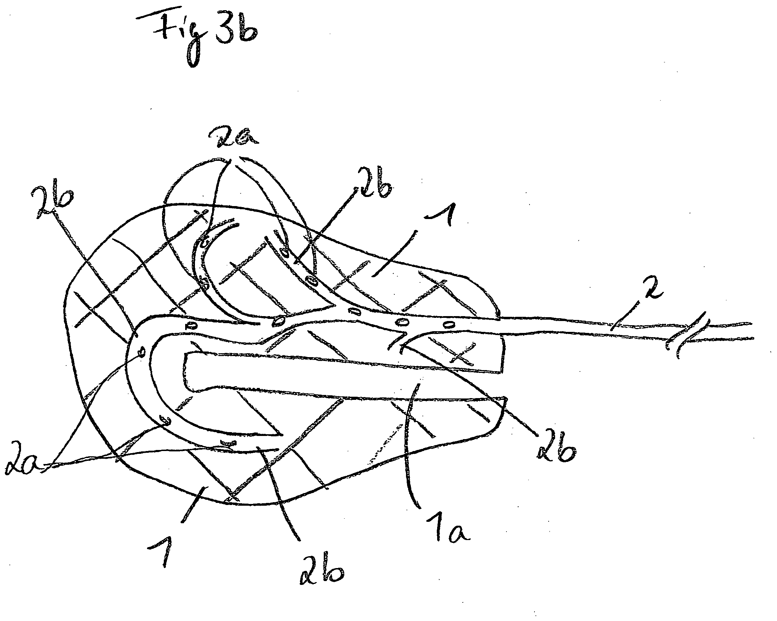

Figur 3b ist eine Längsschnittdarstellung eines Fluidsammelkörpers (1) mit zylindrischem Hohlraum (1a) für einen Führungsstab. Im Fluidsammelelement (1) ist ein schlauchförmiges Fluidkommunikationselement (2) mit Perforationsöffnungen (2a). Das Fluidkommunikationselement ist mit mehreren astförmigen Abzweigungen (2b) verzweigt.

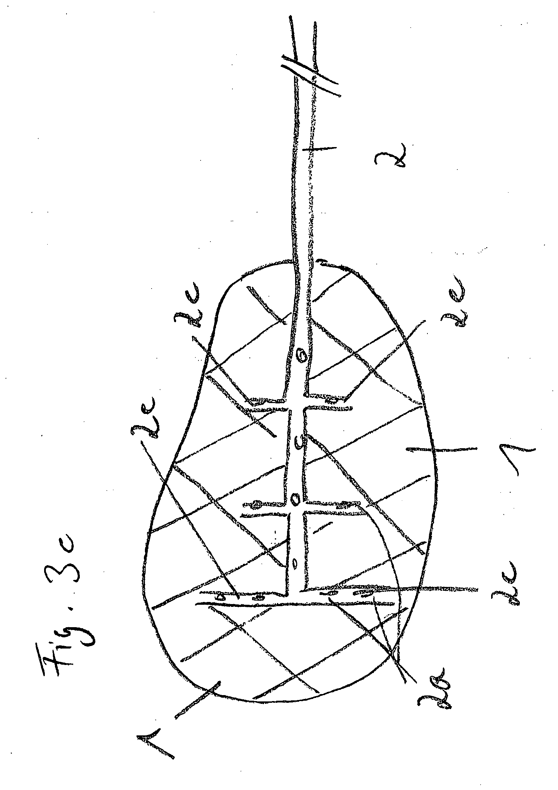

Figur 3c ist eine Längsschnittdarstellung eines Fluidsammelkörpers (1) mit einem schlauchförmigen Fluidkommunikationselement (2) (mit Perforationsöffnungen 2a), welches T-stückartige Abzweigungen (2c) aufweist.
Figur 3c ist eine Längsschnittdarstellung eines Fluidsammelkörpers (1) mit einem schlauchförmigen Fluidkommunikationselement (2) (mit Perforationsöffnungen (2a)) welches in einer Schlaufe (2d) in dem Fluidsammelkörper einliegt. Das Fluidsammelelement tritt über ein Öffnungen (2e) ein und aus und vereinigt sich y-förmig (2f).

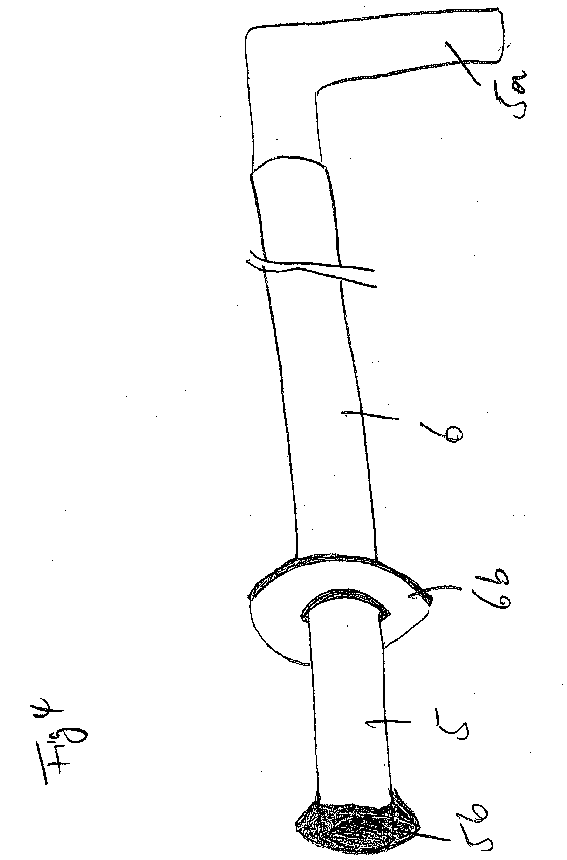

Figur 4 ist eine Darstellung des Führstabes (5) mit verschieblicher Hülse (6). Der Führungsstab (5) hat einen Handgriff (5a). Die Hülse schließt am distalen Ende mit einer Scheibe (6b) ab. Der Führungsstab (5) ist am distalen Ende zur Verringerung eines Perforationsrisikos stempelartig aufgetrieben (5b).

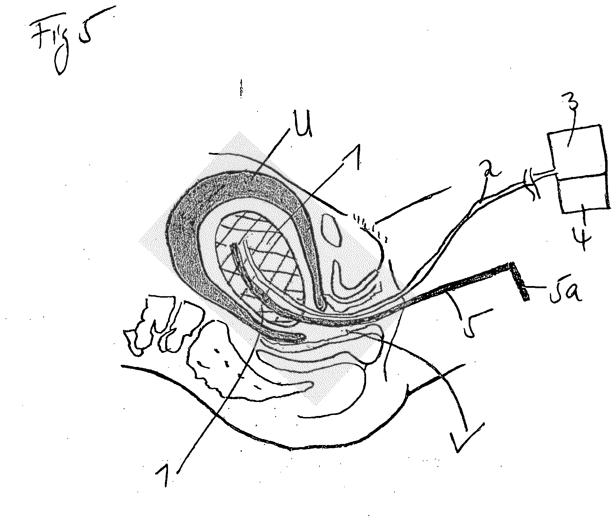

Figur 5 ist eine Längsschnittdarstellung durch den weiblichen Unterleib. Der Uterus (U) ist groß und muskelstark nach der Geburt. Durch die Vagina (V) ist das Fluidsammelelement (1) mit dem Führungsstab (5), der gekrümmt ist und dem natürlichen Verlauf des Zugangsweges angepasst ist, eingeführt worden. Der Führungsstab hat einen Handgriff (5a). Das Fluidsammelelement (1) ist fluidleitend mit dem schlauchförmigen Fluidkommunikationselement (2) verbunden, welches mit dem Sekretauffangbehälter (3) und der Unterdruckpumpe (4) verbunden ist. Es liegt noch kein Unterdruck an.

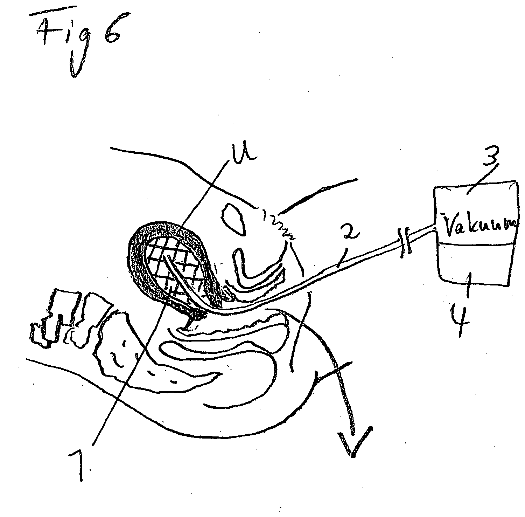

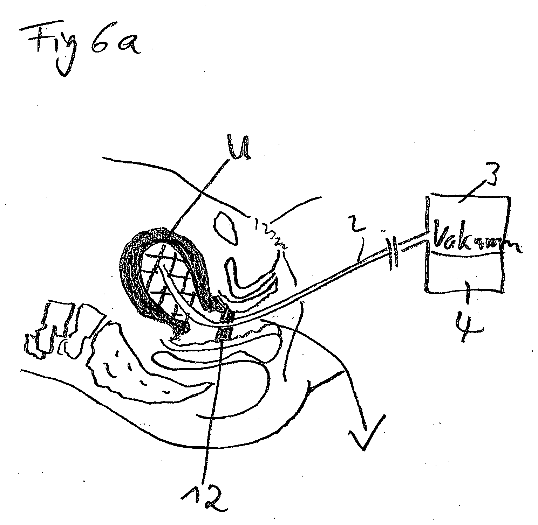

Figur 6 ist ebenso wie Figur 5 eine Längsschnittdarstellung durch den weiblichen Unterleib. Der Führungsstab ist entfernt. Die Unterdruckpumpe (4) erzeugt ein Vakuum in dem Sekretauffangbehälter (3). Dieser ist fluidleitenden mit dem Fluidkommunikationselement (2) verbunden. Das Vakuum wird an das Fluidsammelelement (1) weitergeleitet. Der Uterus (U) ist mit seiner Innenwand an das Fluidsammelelement (1) angesaugt, welches unter dem Sog kollabiert. Mit der Unterdruckpumpe (3) können nach dem Ansaugen des Uterus (U) spezifische Unterdruckmuster genieriert werden und auf die Uteruswand übertragen werden. Vagina (V)
Figur 6a ist die gleiche Längsschnittdarstellung wie Figur 6 durch den weiblichen Unterleib. Zusätzlich zu Figur 6 ist in die Vagina ein Pessar (12) zur besseren Abdichtung eingeführt. Der Führungsstab ist entfernt. Die Unterdruckpumpe (4) erzeugt ein Vakuum in dem Sekretauffangbehälter (3). Dieser ist fluidleitend mit dem Fluidkommunikationselement (2) verbunden. Das Vakuum wird an das Fluidsammelelement (1) weitergeleitet. Der Uterus (U) ist mit seiner Innenwand an das Fluidsammelelement (1) angesaugt, welches unter dem Sog kollabiert. Mit der Unterdruckpumpe (3) können nach dem Ansaugen des Uterus (U) spezifische Unterdruckmuster generiert werden und auf die Uteruswand übertragen werden. Vagina (V)

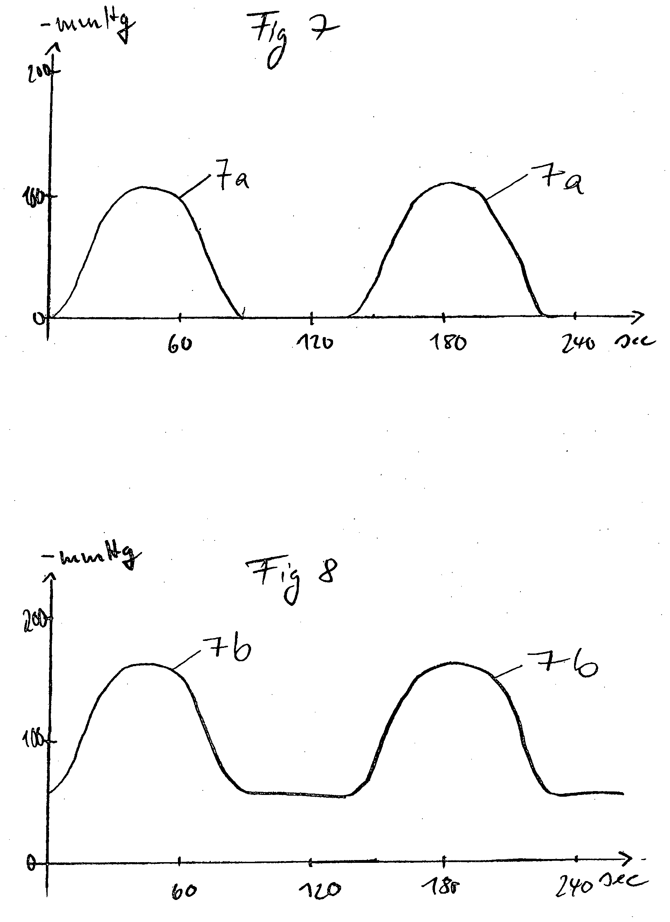

Figur 7 ist die Darstellung eines Unterdruckmuster mit einem Wehen nachempfundenen Muster, welches mit der Unterdruckpumpe erzeugt werden kann. Der glockenförmige sich wiederholende Unterdruckverlauf (7a) besteht in dieser Beispielausführung über ca. 90 sec und pausiert ca. 40 sec. Der maximale Unterdruck liegt bei -100 mmHg. In dem Pausen liegt kein Unterdruck an der Drainage an.
Figur 8 ist die Darstellung eines Unterdruckmusters mit einem Wehen nachempfundenen Muster, welches mit der Unterdruckpumpe erzeugt werden kann. Der glockenförmige sich wiederholende Unterdruckverlauf (7b) besteht in dieser Beispielausführung über ca. 90 sec und pausiert ca. 40 sec. Der maximale Unterdruck liegt bei -150 mmHg. Der minimale Unterdruck, dessen Plateau in den Pausen nicht überschritten wird und den Basisunterdruck darstellt liegt bei -50 mmHg. Das bedeutet, dass bei diesem Muster immer ein Unterdruck an der Drainage anliegt.

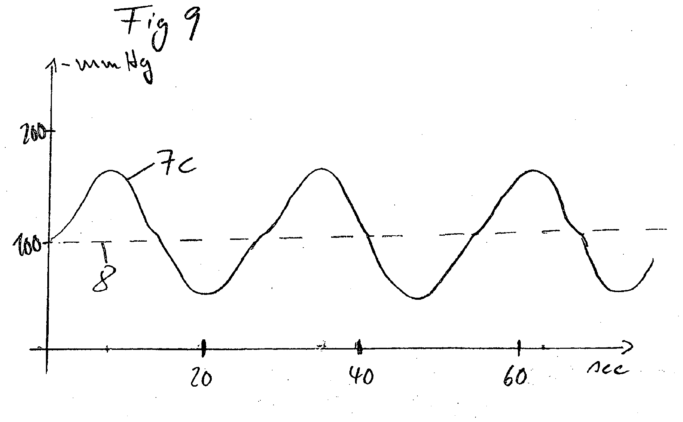

Figur 9 ist die Darstellung eines Unterdruckmusters, welches mit der Unterdruckpumpe erzeugt werden kann, mit undulierendem Unterdruckverlauf (7). Der Höchste und niedrigste Unterdruck pendeln um einen Mittleren Unterdruck (8) in dieser beispielhaften Ausführung von 100 mmHg.

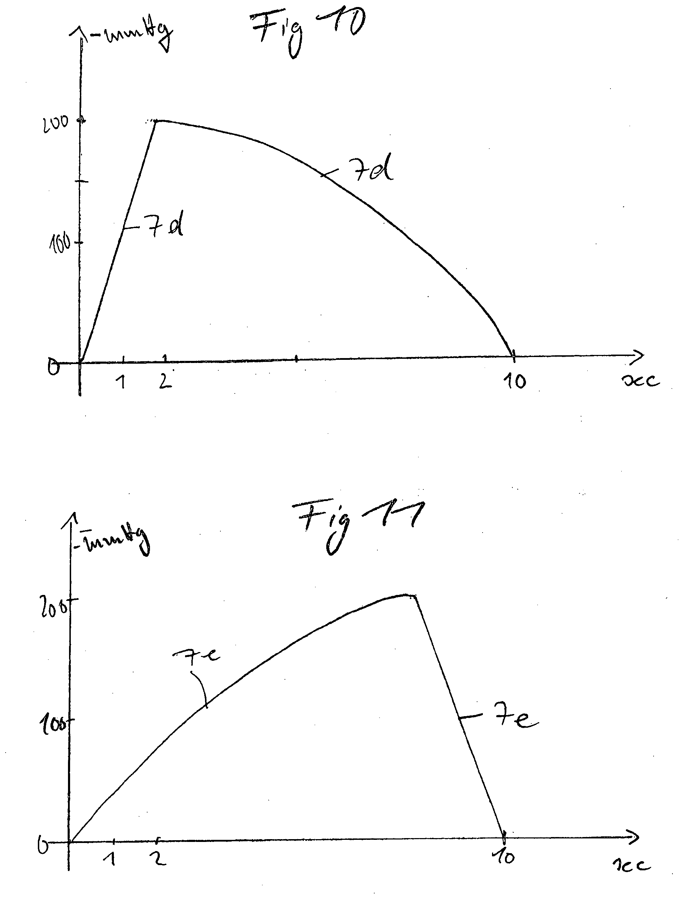

Figur 10 ist die Darstellung eines sägezahnartigen Unterdruckverlaufes (7d), welches mit der Unterdruckpumpe erzeugt werden kann. Innerhalb von 2 sec wird ein Unterdruck von 200 mmHg erzeugt, hiernach fällt der Unterdruck innerhalb von 8 sec auf 0 mmHg ab.
Figur 11 ist die Darstellung eines sägezahnartigen Unterdruckverlaufes (7e), welches mit der Unterdruckpumpe erzeugt werden kann. Innerhalb von 8 sec wird ein Unterdruck von 200 mmHg erzeugt, hiernach fällt der Unterdruck innerhalb von 2 sec auf 0 mmHg ab.

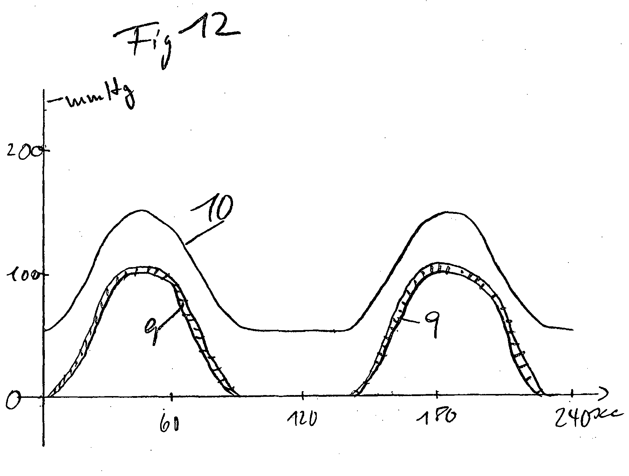

Figur 12 ist die Darstellung eines wehenartigen Unterdruckverlaufes (10) mit einem Plateau in der Wehenpause bei -50 mmHg und einem maximalen Unterdruck von -150. Dieser Unterdruckverlauf ist synchron mit der abgeleiteten Wehentätigkeit (9). Die Unterdruckpumpe nimmt das Signal der Wehentätigkeit (9) auf und synchronisiert den Verlauf des Unterdrucks. Die Wehentätigkeit kann elektromyographisch abgeleitet werden und der Pumpe als Steuerungssignal zugeleitet werden.

## Patentansprüche

1. Drainage zur intrauterinen Unterdrucktherapie,
- welche aus einem offenporigen mit mindestens einem Fluidkommunikationselement (2) verbundenen Fluidsammelelement (1) besteht, welches in der Längsachse oder parallel der Längsachse, über die gesamte oder teilweise Länge der Achse, einen zylindrischen röhrenartigen Hohlraum (1a) mit einer Eintrittsöffnung am proximalen Ende aufweist, in welchen ein entfernbarer Führungsstab (5) oder ein Endoskop (11) zur transvaginalen Platzierung eingebracht ist - und welche fluidleitend mit einer elektronischen Unterdruckpumpe (4) verbunden ist, die intrauterine Unterdruckmuster (7, 7a, 7b) an der inneren Gebärmutterwand erzeugen kann, die denen von Geburtswehen und/oder postpartalen Gebärmutterkontraktionen ähnlich sind, wobei sich auf dem Führungsstab (5) oder dem Endoskop (11) eine verschiebliche Pusherhülse (6) befindet, die an ihrem distalen Ende trichterförmig aufgeweitet ist und/oder mit einer Schiebeplatte (6b) abschließt.

2. Drainage und Unterdruckpumpe zur intrauterinen Unterdrucktherapie nach Anspruch eins, wobei das Fluidsammelelement (1) aus einem offenporigen Polyurethanschaum und/oder einer offenporigen Folie oder einer Kombination aus verschiedenen offenporigen Materialien besteht, welcher eine Porengröße von 100 µm -2000 µm aufweisen und eine zylindrische Formgebung mit einem Durchmesser in der Querachse von 1 cm -15 cm und in der Längsachse von 2 cm-30 cm aufweisen.

3. Drainage und Unterdruckpumpe zur intrauterinen Unterdrucktherapie nach einem der vorherigen Ansprüche, wobei das Fluidsammelelement (1)als intrauteriner Medikamententräger dient und/oder mit lokal blutstillenden Medikamenten ausgerüstet ist.

4. Drainage und Unterdruckpumpe zur intrauterinen Unterdrucktherapie nach einem der vorherigen Ansprüche, wobei das Fluidsammelelement (1) eine Kompressionsstabilität zwischen 10% und 90% unter einem Vakuum zwischen 20 mmHg und 200 mmHg aufweist.

5. Drainage und Unterdruckpumpe zur intrauterinen Unterdrucktherapie nach einem der vorherigen Ansprüche, wobei das Fluidkommunikationselement (2) aus einem unterdruckstabilen Schlauch besteht, welcher transparent ist und einen Durchmesser von 2 mm - 20 mm und eine Länge von 30 cm - 120 cm aufweist und in der Längsachse oder parallel zur Längsachse des Fluidsammelelementkörpers (1) oder als eine Schlaufe (2d) und/oder verzweigt in demselben einliegt.

6. Drainage und Unterdruckpumpe zur intrauterinen Unterdrucktherapie nach einem der vorherigen Ansprüche, wobei der Führungsstab (5) aus Kunststoff oder Metall besteht und 20 cm bis 120 cm lang ist und einen Durchmesser von 2 mm bis 25 mm hat oder wobei ein Endoskop (11) als Führungsstab dient.

7. Drainage und Unterdruckpumpe zur intrauterinen Unterdrucktherapie nach einem der vorherigen Ansprüche, wobei die Unterdruckpumpe (4)einen Unterdruck von 20 mmHg -200 mmHg aufbauen kann und spezifische Sogmuster (7) und/oder kontinuierliche und/oder intermittierende und/oder undulierende und/oder sägeblattartige Saugmuster (7) erzeugen kann.

8. Drainage und Unterdruckpumpe zur intrauterinen Unterdrucktherapie nach einem der vorherigen Ansprüche, wobei die Unterdruckpumpe (4) bei einem Totvolumen von 200 ml bis 500 ml bei einem Sogabfall innerhalb von 2 sec den eingestellten maximalen Unterdruck erzeugen kann oder wobei sie den maximalen Unterdruck kontinuierlich steigernd innerhalb von 10 - 120 sec erzeugen kann.

9. Drainage und Unterdruckpumpe zur intrauterine Unterdrucktherapie nach einem der vorherigen Ansprüche, wobei die Unterdruckpumpe (4) mit einer Steuereinheit ausgerüstet, welche Unterdruckmesssignale von Messfühlern am fluidleitenden System der intrauterinen Drainage und/oder Muskelkontraktionspotentiale der Gebärmutter automatisch verarbeitet und den Unterdruck und/oder das Sogmuster (7) regelt und/oder den Unterdruck simultan zu den Kontraktionen der Gebärmutter anpasst und/oder die Unterdruckerzeugung mit den Kontraktionen der Gebärmutter synchronisiert.

## Claims

1. A drainage system for intrauterine vacuum therapy,
- which consists of an open-cell fluid-collecting element (1), which is connected to at least one fluid-communicating element (2) and has, in the longitudinal axis or parallel to the longitudinal axis, over the entire or partial length of the axis, a cylindrical tubular cavity (1a) with an entry opening at the proximal end, into which entry opening a removable guide rod (5) or an endoscope (11) for transvaginal placement has been introduced,
- and which is fluid-conductively connected to an electronic vacuum pump (4), which can generate, on the inner uterine wall, intrauterine vacuum patterns (7, 7a, 7b) which are similar to those resulting from labour contractions and/or postpartum uterine contractions,
wherein a movable pusher sheath (6) is located on the guide rod (5) or endoscope (11) which is widened in the form of a funnel at its distal end and/or is terminated with a pusher plate (6b).

2. The drainage system and vacuum pump for intrauterine vacuum therapy according to claim 1, wherein the fluid-collecting element (1) consists of an open-pore polyurethane foam and/or an open-cell film or a combination of different open-cell materials, which have a pore size of 100 µm to 2000 µm, and which fluid-collecting element has a cylindrical shape with a diameter of 1 cm to 15 cm on the transverse axis and of 2 cm to 30 cm on the longitudinal axis.

3. The drainage system and vacuum pump for intrauterine vacuum therapy according to one of the preceding claims, wherein the fluid-collecting element (1) acts as an intrauterine medication carrier and/or is equipped with locally haemostatic medications.

4. The drainage system and vacuum pump for intrauterine vacuum therapy according to one of the preceding claims, wherein the fluid-collecting element (1) has a compression stability of between 10% and 90% subject to a vacuum of between 20 mmHg and 200 mmHg.

5. The drainage system and vacuum pump for intrauterine vacuum therapy according to one of the preceding claims, wherein the fluid-communication element (2) consists of a tube which is stable under vacuum, is transparent and has a diameter of 2 mm to 20 mm and a length of 30 cm to 120 cm and exists in the longitudinal axis or parallel to the longitudinal axis of the fluid-collecting element body (1) within the same or as a loop (2d) and/or in a branched form.

6. The drainage system and vacuum pump for intrauterine vacuum therapy according to one of the preceding claims, wherein the guide rod (5) is made of plastic or metal and is 20 cm to 120 cm long and has a diameter of 2 mm to 25 mm, or wherein an endoscope (11) acts as the guide rod.

7. The drainage system and vacuum pump for intrauterine vacuum therapy according to one of the preceding claims, wherein the vacuum pump (4) can generate a vacuum of 20 mmHg to 200 mmHg and can generate specific suction patterns (7) and/or continuous and/or intermittent and/or undulating and/or sawtooth-like suction patterns (7).

8. The drainage system and vacuum pump for intrauterine vacuum therapy according to one of the preceding claims, wherein the vacuum pump (4) can generate the set maximum vacuum at a dead volume of 200 ml to 500 ml with a suction drop within 2 s, or wherein it can generate the maximum vacuum continuously rising within 10 s to 120 s.

9. The drainage system and vacuum pump for intrauterine vacuum therapy according to one of the preceding claims, wherein the vacuum pump (4) is equipped with a control unit which automatically processes vacuum measurement signals from measuring sensors on the fluid-conducting system of the intrauterine drainage system and/or muscle contraction potentials of the uterus, and regulates the vacuum and/or the suction pattern (7) and/or adapts the vacuum simultaneously to the uterine contractions and/or synchronises the vacuum generation with the uterine contractions.

## Revendications

1. Drainage pour thérapie intra-utérine par dépression,
- qui se compose d'un élément collecteur de fluide (1) à pores ouverts qui est relié à au moins un élément de communication de fluide (2) et présente, dans l'axe longitudinal ou parallèlement à l'axe longitudinal, sur toute la longueur ou sur une longueur partielle de l'axe, une cavité (1a) tubulaire cylindrique comportant une ouverture d'entrée à l'extrémité proximale, dans laquelle est disposée une tige de guidage (5) amovible ou un endoscope destiné à une mise en place transvaginale,
- et qui est relié par communication fluidique à une pompe à dépression (4) électronique susceptible de produire des configurations de dépression intra-utérine (7, 7a, 7b) sur la paroi interne de l'utérus qui sont semblables à celles des contractions de l'accouchement et/ou postpartales,
une gaine de poussée (6) déplaçable se trouvant sur la tige de guidage (5) ou l'endoscope (11) et étant élargie en forme d'entonnoir à son extrémité distale et/ou se terminant par une plaque de coulissement (6b).

2. Drainage et pompe à dépression pour thérapie intra-utérine par dépression selon la revendication 1, dans lesquels l'élément collecteur de fluide (1) se compose d'une mousse de polyuréthane à pores ouverts et/ou d'une feuille à pores ouverts ou d'une combinaison de divers matériaux à pores ouverts, lesquels présentent des pores d'une taille allant de 100 µm à 2000 µm, et lequel élément collecteur de fluide présente une conformation cylindrique présentant un diamètre allant de 1 cm à 15 cm dans l'axe transversal et allant de 2 cm à 30 cm dans l'axe longitudinal.

3. Drainage et pompe à dépression pour thérapie intra-utérine par dépression selon l'une des revendications précédentes, dans lesquels l'élément collecteur de fluide (1) sert de support de médicament intra-utérin et/ou est équipé de médicaments localement hémostatique.

4. Drainage et pompe à dépression pour thérapie intra-utérine par dépression selon l'une des revendications précédentes, dans lesquels l'élément collecteur de fluide (1) présente une stabilité à la compression comprise entre 10 % et 90 % sous une dépression comprise entre 20 mmHg et 200 mmHg.

5. Drainage et pompe à dépression pour thérapie intra-utérine par dépression selon l'une des revendications précédentes, dans lesquels l'élément de communication de fluide (2) se compose d'un tuyau stable à la dépression qui est transparent et présente un diamètre allant de 2 mm à 20 mm et une longueur allant de 30 cm à 120 cm et qui se présente dans l'axe longitudinal ou parallèle audit axe longitudinal du corps de l'élément collecteur de fluide (1) ou sous la forme d'une boucle (2d) et/ou sous une forme ramifiée dans ce dernier.

6. Drainage et pompe à dépression pour thérapie intra-utérine par dépression selon l'une des revendications précédentes, dans lesquels la tige de guidage (5) se compose de plastique ou de métal, fait de 20 cm à 120 cm de longueur et présente un diamètre de 2 mm à 25 mm, ou dans lesquels un endoscope (11) sert de tige de guidage.

7. Drainage et pompe à dépression pour thérapie intra-utérine par dépression selon l'une des revendications précédentes, dans lesquels la pompe à dépression (4) peut produire une dépression allant de 20 mmHg à 200 mmHg et peut réaliser des configurations d'aspiration spécifiques (7) et/ou des configurations d'aspiration (7) continues et/ou intermittentes et/ou ondulées et/ou en dents de scie.

8. Drainage et pompe à dépression pour thérapie intra-utérine par dépression selon l'une des revendications précédentes, dans lesquels la pompe à dépression (4) peut produire, à un volume mort allant de 200 ml à 500 ml, en présence d'une chute d'aspiration, la dépression maximale réglée, en l'espace de 2 s ou dans lesquels elle peut produire la dépression maximale en montée continue en l'espace de 10 à 120 s.

9. Drainage et pompe à dépression pour thérapie intra-utérine par dépression selon l'une des revendications précédentes, dans lesquels la pompe à dépression (4) est équipée d'une unité de commande qui traite automatiquement des signaux de mesure de dépression en provenance de capteurs de mesure présents au niveau du système conducteur de fluide du drainage intra-utérin et/ou des potentiels de contractions musculaires de l'utérus et qui règle la dépression et/ou la configuration d'aspiration (7) et/ou adapte la dépression simultanément aux contractions de l'utérus et/ou synchronise la production de la dépression avec les contractions de l'utérus.
